# EUROPEAN PATENT SPECIFICATION

(11) **EP 0 979 824 B1**
(45) Date of publication and mention of the grant of the patent: **06.10.2004**
(21) Application number: 99115244.8
(22) Date of filing: 02.08.1999
(51) Int. Cl.: C07D 491/08, C07D 211/70, C07D 211/52, C07D 211/74

(54) **Process for the preparation of substituted piperidine-epoxides**
Verfahren zur Herstellung von substituierte piperidinepoxiden
Procédé pour la préparation des epoxides de pipéridine substitués

(30) Priority: 10.08.1998 EP 98114975
(43) Date of publication of application: 16.02.2000
(73) Proprietor: F. HOFFMANN-LA ROCHE AG, 4070 Basel (CH)
(72) Inventor: Gueller, Rolf, 5027 Herznach (CH); Lohri, Bruno, 4153 Reinach (CH); Schmid, Rudolf, 4057 Basle (CH)
(74) Representative: Witte, Hubert, Dr.

(56) References cited:
- WO-A-97/09311
- SUNDBERG R J ET AL: "Synthesis and gramine alkylation of some 3-piperidones. Synthetic route to 2-(3-indolylmethyl)-4-piperidineacetic acid derivatives" J. ORG. CHEM. (JOCEAH);1971; VOL.36 (17); PP.2471-80, XP002115962 Univ. Virginia;Dep. Chem.; Charlottesville; Va.
- NAGAI Y ET AL: "Studies on psychotropic agents. VI. Synthesis of 1-methylspiro[6-fluoroindan-1,3'-pyrrolidi n]-3-one and related compounds" CHEM. PHARM. BULL. (CPBTAL,00092363);1980; VOL.28 (5); PP.1387-93, XP002115963 Dainippon Pharm. Co., Ltd.;Res. Lab.; Suita; Japan
- HERSHENSON F M ET AL: "Reaction of 1-carbamoyl-3,4-epoxy-4-phenylpiperidines with Lewis acids" SYNTH. COMMUN. (SYNCAV,00397911);1981; VOL.11 (8); PP.615-25, XP002115964 G. D. Searle and Co.;Dep. Med. Chem.; Chicago; 60680; IL; USA (US)
- ROUSSELET G ET AL: "Copper-catalyzed olefin epoxidation by dioxygen or amine N-oxide" TETRAHEDRON LETT. (TELEAY,00404039);1996; VOL.37 (47); PP.8497-8500, XP002115965 CNRS;Lab. Rech. Org.; Paris; F-75231; Fr. (FR)

## Description

The invention relates to a novel process for the preparation of substituted piperidine-epoxides. More particularly the invention relates to the preparation of compounds of the formula 1 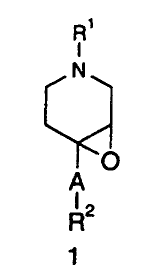 and salts thereof, wherein
- A: is arylene;
- R¹: is -C*HR⁴R⁵;
- R²: is -O-alkyl, -O-cycloalkyl, -O-alkenyl, -O-aryl, -O-aralkyl, - O-aralkoxyalkyl, -O-alkylsulfonyl, -O-arylsulfonyl, chlorine, bromine or iodine;
- R⁴: is aryl;
- R⁵: is alkyl, cycloalkyl, aryl, alkoxyalkyl or hydroxyalkyl;
and C* is an asymmetric carbon atom.

The compounds of formula 1 are new and can be used as chiral building blocks in the preparation of renin inhibitors especially trisubstituted renin inhibitors as disclosed in WO 97/09311 e.g. (3R,4S,5S)-4-[4-(3-benzyloxy-propoxy)-phenyl]-5-ethoxy-3-(naphthalen-2-ylmethoxy)-piperidine. The syntheses of optically active renin inhibitors via resolution of racemates as disclosed in WO 97/09311 results in a considerable loss of product. The present invention provides a process avoiding the disadvantages of the above process.

According to the present invention the compounds of formula 1 above or salts thereof can be prepared by a process characterised in that it comprises
a) epoxidation of a compound of the formula 2 or a salt thereof 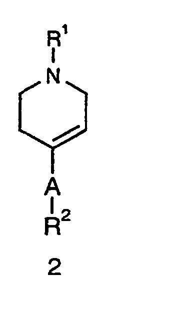 in which formula
   R¹, R² and A are defined as above;
   and
b) optionally isolation of the desired stereoisomer.

The term "alkyl" means alone or in combination a branched or unbranched alkyl group containing 1 to 8 carbon atoms, preferred 1 to 6 carbon atoms. Examples for branched or unbranched C₁-C₈ alkyl groups are methyl, ethyl, propyl, isopropyl, butyl, isobutyl, tert-butyl, the isomeric pentyls, the isomeric hexyls, the isomeric heptyls, the isomeric octyls and preferred ethyl, n-propyl, and isopropyl and particularly preferred methyl.

The term "cycloalkyl" means alone or in combination a cycloalkyl cycle with 3 to 8 carbon atoms and preferred a cycloalkyl cycle with 3 to 6 carbon atoms. Examples for C₃-C₈ cycloalkyl are cyclopropyl, methyl-cyclopropyl, dimethyl-cyclopropyl, cyclobutyl, methyl-cyclobutyl, cyclopentyl, methylcyclopentyl, cyclohexyl, methyl-cyclohexyl, dimethyl-cyclohexyl and cycloheptyl.

The term "alkenyl" means alkenyl groups of 2 to 8 carbon atoms. Examples of alkenyl groups include vinyl, allyl, isopropenyl, pentenyl, hexenyl, heptenyl, cyclopropenyl, cyclobutenyl, cyclopentenyl, cyclohexenyl, 1-propenyl, 2-butenyl, 2-ethyl-2-butenyl, and the like, preferably allyl.

The term "aryl" means alone or in combination a phenyl or a naphthyl group which can be substituted by one or several substituents chosen from alkyl, cycloalkyl, alkoxy, halogen, carboxy, alkoxycarbonyl, hydroxy, amino, nitro, trifluoromethyl and the like. Examples for aryl are phenyl, tolyl, methoxyphenyl, fluorophenyl, chlorophenyl, hydroxyphenyl, trifluoromethylphenyl, 1-naphthyl and 2-naphthyl.

The term "arylene" means alone or in combination a phenylene or a naphthylene group which can be additionally substituted by one or several substituents chosen from alkyl, cycloalkyl, halogen, nitro, alkoxy, hydroxy, amino, preferably alkyl, halogen and nitro. Examples for arylene are ortho-phenylene, meta-phenylene, para-phenylene, the tolylenes, methoxyphenylenes, fluorophenylenes, chlorophenylenes and naphthylenes. Preferred are phenylene, wherein the substituents of the phenylene which are defined by formula 1 are placed ortho, meta or preferred para to one another and wherein one or several additional substituents chosen from alkyl, halogen and nitro can be present at the arylene cyclus. Especially preferred substituents are methyl, chloro and nitro. Particularly preferred is unsubstituted phenylene and especially unsubstituted para phenylene.

The term "alkoxy" means alone or in combination the group -O-alkyl, wherein alkyl is defined as before. Examples are ethoxy, n-propyloxy, and iso-propyloxy. Preferred is methoxy.

The term "alkoxyalkyl" means alone or in combination an alkyl group, wherein a hydrogen is substituted by an alkoxy group. Examples are methoxymethyl, ethoxymethyl and 2-methoxyethyl. Particularly preferred is methoxymethyl.

The term "hydroxyalkyl" means alone or in combination an alkyl group, wherein a hydrogen is substituted by an hydroxy group. Examples are hydroxymethyl, 1-hydroxyethyl and 2-hydroxyethyl. Preferred is hydroxymethyl.

The term "aralkyl" means alone or in combination an alkyl group, wherein a hydrogen is substituted by an aryl group. A preferred example is benzyl.

The term "aralkoxyalkyl" means alone or in combination an alkyl group, wherein a hydrogen is substituted by an alkoxy group in which a hydrogen is substituted by an aryl group. A preferred example for aralkoxyalkyl is 3-(2-methoxy-benzyloxy)-propyl.

The term "alkylsulfonyl" means alone or in combination a sulfonyl group which is substituted by an alkyl group. The alkyl group can be substituted by halogen. Preferred examples are methylsulfonyl and trifluoromethylsulfonyl.

The term "arylsulfonyl" means alone or in combination a sulfonyl group which is substituted by an aryl group. Preferred is the tosyl group.

The term "salts" means compounds which are formed by reaction of compounds of formula 1 with inorganic acids such as hydrochloric acid, hydrobromic acid, sulfuric acid, nitric acid, phosphoric acid, and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. The term salts includes solvates and particularly hydrates of such salts.

The term "halogen" means fluorine, chlorine, bromine, iodine, preferably chlorine and bromine. Most preferred is chlorine.

The term "anion" means an atom, a group of atoms or a molecule with negative charge. This charge can be a single or a multiple charge. Examples of anions are the halogen anions, SO₄²⁻, PO₄³⁻. Particularly preferred is the Cl⁻ anion.

The term "asymmetric carbon atom (C*)" means a carbon atom with four different substituents. According to the Cahn-Ingold-Prelog-Convention the asymmetric carbon atom can be of the " R " or " S " configuration. A preferred example for an asymmetric carbon atom C* is showm in the formula 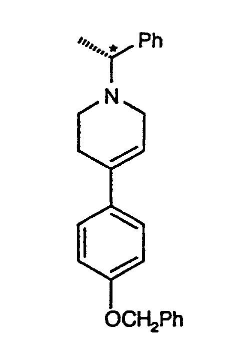 wherein the asymmetric carbon atom C* is of the R configuration.

The term "-O-" in groups such as -O-alkyl, -O-cycloalkyl, -O-alkenyl,-O-aryl, -O-benzyl, -O-aralkoxyalkyl, -O-alkylsulfonyl, -O-arylsulfonyl, means an oxygen with a free valence. For example -O-alkyl means alkoxy and -O-cycloalkyl means cycloalkoxy.

In a preferred aspect, the above invention is concerned with the preparation of compounds of the formula 1, wherein R⁵ is alkyl or cycloalkyl and R¹, R² and A are defined as above.

Also preferred is the above process, wherein R⁴ is unsubstituted phenyl or substituted phenyl and, wherein the substituents of phenyl are one or several chosen from alkyl, halogen or nitro, preferably methyl or chloro. In a particularly preferred embodiment of the above process R⁴ is unsubstituted phenyl and R¹, R² and A are as defined above.

Particularly preferred is the above process, wherein R⁴ is phenyl, particularly preferred unsubstituted phenyl and R⁵ is methyl and R¹, R² and A are defined as above.

Also preferred is the above process, wherein A is substituted or unsubstituted ortho, meta or para phenylene, wherein the substituents of the phenylene which are defined by formula 1 are placed ortho, meta or para to one another. The para position is preferred. The substituted phenylene has one to four additional substituents chosen from alkyl, halogen and nitro. Particularly preferred is the above process, wherein A is unsubstituted phenylene and especially unsubstituted para phenylene.

Preferred is also the process of the present invention, wherein R² is -O-alkyl, -O-cycloalkyl, -O-aryl, or -O-aralkyl, preferably -O-benzyl and -O-methyl. Particularly preferred is -O-benzyl.

In a preferred embodiment of the present invention a compound of formula 2 is reacted with a halogen or a halogen delivering agent such as N-bromine compounds. Preferred examples are bromine, N-bromosuccinimide, dibromoisocyanurate and 1,3-dibromo-5,5-dimethylhydantoin. Particularly preferred is bromine.

The preparation of compounds of formula 2 by a process comprising the reaction of compounds of formula 3 or 4 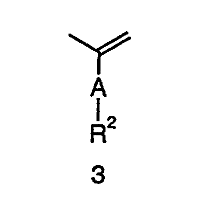 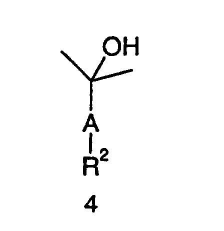 with compounds of the formula R¹-NH₂ or a salt thereof, preferably in the presence of formaldehyde or a chemical equivalent thereof, wherein R¹, R² and A are defined as above.

Another preferred aspect of the present invention is the isolation of the desired stereoisomer of a compound of formula 1 or a salt thereof, preferably by crystallisation. Particularly preferred is the crystallisation of the free compound of formula 1 and preferably in the presence of ethyl acetate.

Also preferred is the above process followed by a reaction with a metal alcoholate such as potassium t-butoxide, aluminium isopropoxide, titanium (IV) t-butoxide, with a lithium amide such as lithium diisopropylamide or with an organolithium compound such as phenyllithium, sec-butyllithium or methyllithium to form a compound of the general formula 5 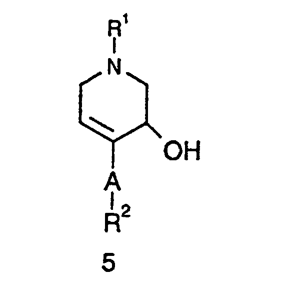 or a salt thereof. Moreover, a preferred aspect of the above process is the reaction of a compound of the formula 1 or a salt thereof, with phenyllithium. Particularly preferred is the above process, wherein the desired stereoisomer of a compound of the formula 1 reacts with phenyllithium.

Another preferred aspect of the present invention is the transformation of compounds of the formula 1 to renin inhibitors, especially trisubstituted renin inhibitors and particularly preferred to (3R,4S,5S)-4-[4-(3-benzyloxy-propoxy)-phenyl]-5-ethoxy-3-(naphthalen-2-ylmethoxy)-piperidine, wherein this transformation is effected by reaction with phenyllithium, alkylation of the 3-hydroxy function, hydroboration of the ether compound formed and subsequent basic oxidative working-up, in order to transform of the intermediate carboboranes into the secondary alcohols, reorganization of protective groups, removal of the N-phenylethyl and the O-benzyl function and re-introduction of a N-Boc protective group, selective functionalization of the phenolic function, alkylation of the secondary hydroxy function of the piperidine ring and removal of the Boc-protective group. Particularly preferred is the above process, wherein a compound of formula 1 or a salt thereof is converted to (3R,4S,5S)-4-[4-(3-benzyloxy-propoxy)-phenyl]-5-ethoxy-3-(naphthalen-2-ylmethoxy)-piperidine characterised in that
a) (1R, 6R)-6-(4-benzyloxy-phenyl)-3-[(R)-1-phenyl-ethyl]-7-oxa-3-azabicyclo [4.1.0] heptane is transformed into (3S)-4-(4-benzyloxy-phenyl)-1-[(1R)-phenyl-ethyl]-1,2,3,6-tetrahydro-pyridin-3-ol by reaction with phenyllithium;
b) reaction with sodium hydride and ethyl iodide yields (3S)-4-(4-benzyloxy-phenyl)-3-ethoxy-1-[(1R)-phenyl-ethyl]-1,2,3,6-tetrahydropyridine;
c) hydroboration with sodium borohydride and boron trifluoride etherate followed by reaction with potassium hydroxide and hydrogen peroxide yields (3R,4R,5S)-(4-benzyloxy-phenyl)-5-ethoxy-1-[(1R)-phenyl-ethyl]-piperidin-3-ol);
d) hydrogenolysis yields (3R,4R,5S)-5-ethoxy-4-(4-hydroxy-phenyl)-piperidin-3-ol;
e) reaction with di-tert-butyldicarbonate and sodium hydrogencarbonate followed by addition of NaOH yields (3R,4R,5S)-5-ethoxy-3-hydroxy-4-(4-hydroxy-phenyl)-piperidine-1-carboxylic acid tert-butylester;
f) treatment with 3-bromo-propoxymethyl-benzene and potassium carbonate yields (3R,4R,5S)-4-[4-(3-benzyloxy-propoxy)-phenyl]-5-ethoxy-3-hydroxy-piperidine-1-carboxylic acid tert-butylester;
g) reaction with 2-bromomethyl-naphthalene and sodium hydride yields (3R,4S,5S)-4-[4-(3-benzyloxy-propoxy)-phenyl]-5-ethoxy-3-(naphthalen-2-ylmethoxy)-piperidine-1-carboxylic acid tert-butylester;
h) reaction with hydrochloric acid yields (3R,4S,5S)-4-[4-(3-benzyloxypropoxy)-phenyl]-5-ethoxy-3-(naphthalen-2-ylmethoxy)-piperidine.

Compounds of the formula 1 and their salts are new and also part of the present invention, wherein R¹, R² and A are defined as above. A preferred compound is (1R, 6R)-6-(4-benzyloxy-phenyl)-3-[(R)-1-phenyl-ethyl]-7-oxa-3-aza-bicyclo [4.1.0] heptane.

Compounds of the formula 2 and their salts are new and also part of the present invention, wherein R¹, R² and A are defined as above. Particularly preferred is (R)-4-(4-benzyloxy-phenyl)-1-(1-phenyl-ethyl)-1,2,3,6-tetrahydro-pyridine and salts thereof.

Furthermore, (R)-4-(4-benzyloxy-phenyl)-1-(1-phenyl-ethyl)-piperidin-4-ol and salts thereof are new and also a part of the present invention.

Moreover, compounds of the formula 5 and their salts are new and a part of the present invention, preferably (3S)-4-(4-benzyloxy-phenyl)-1-[(1R)-phenyl-ethyl]-1,2,3,6-tetrahydro-pyridin-3-ol.

The invention also relates to the use of a compound of formula 1 in the preparation of renin inhibitors, preferably (3R,4S,5S)-4-[4-(3-benzyloxy-propoxy)-phenyl]-5-ethoxy-3-(naphthalen-2-ylmethoxy)-piperidine, wherein R¹, R² and A are defined as described before. The transformation of compounds of formula 1 into renin inhibitors, especially into (3R,4S,5S)-4-[4-(3-benzyloxy-propoxy)-phenyl]-5-ethoxy-3-(naphthalen-2-ylmethoxy)-piperidine can be performed as described above.

Furthermore, the invention also relates to compounds as obtained by the above processes.

In more detail, the process of the invention may be described as follows:

Epoxidation of a compound of formula 2 or a salt thereof and optionally isolation of the desired stereoisomer: 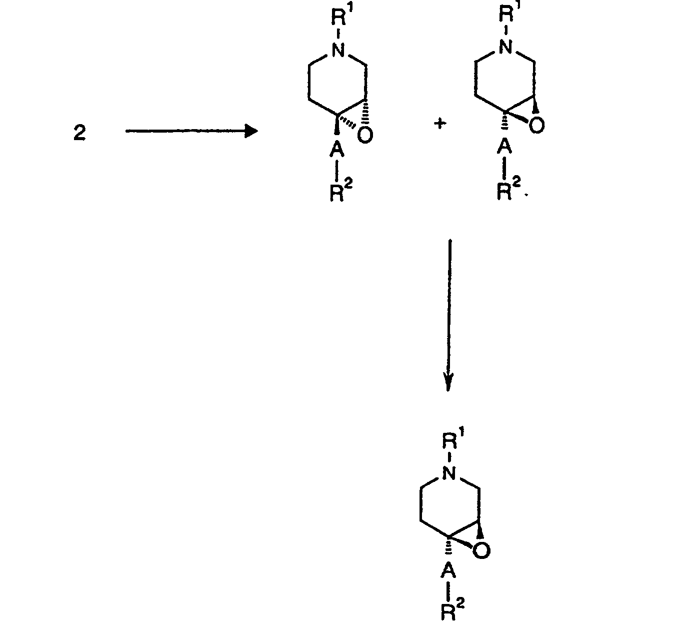 wherein R¹, R² and A are defined as before.

A compound of the formula 2 can be reacted with compounds which are known for use in epoxidation reactions. Examples for such reagents are halogens and organic bromo-compounds such as N-bromosuccinimide, dibromoisocyanurate and 1,3-dibromo-5,5-dimethylhydantoin. Preferred is bromine, especially in the presence of an acid, preferably HBr and chemical equivalents thereof.

Inert solvents taken alone or in combination can be used, particularly, solvents which are known for their utilisation in epoxidation reactions. Examples of such solvents are straight or cyclic ethers dimethylether, diethylether, tetrahydrofuran and monoglyme or diglyme alone or in such a combination that a sufficient miscibility with water is given. A preferred solvent is dioxane.

Preferred is the above reaction in the presence of an acid. Examples of such acids are optically active or inactive acids such as the hydrohalic acids, sulfonic acids and H₂SO₄. Particularly preferred is HBr.

In general the above reaction can be performed in a wide pH range. Preferred is a pH range from about 1 to 4 and particularly preferred is a pH range from about 1,5 to 3.

A temperature range of from about -20°C to the boiling point of the solvent is suitable for the reaction of the present invention. The preferred temperature range is between about -20°C to about 20°C preferably from about 0°C to about 5°C.

In a preferred aspect, the above reaction is followed by addition of a base such as NaOH, KOH, or a nitrogen-base such as triethylamine. Preferred is the use of NaOH or KOH. The temperature range for the addition of the base is between -20°C and the boiling point of the solvent. Preferred is a temperature range between -20°C and 20°C. Particularly preferred is the addition of the base between 0°C and 5°C. In case the epoxidising agent reacts with a compound of the formula 2 without addition of an acid, the epoxide can be obtained without using a base.

According to the above process compounds of formula 1 are formed as a mixture of stereoisomers and particularly as a mixture of diastereomers, or only one of the diastereomers is formed. In a preferred aspect one of the diastereomers is formed preferably.

In a preferred embodiment of the invention (R)-4-(4-benzyloxy-phenyl)-1-(1-phenyl-ethyl)-1,2,3,6-tetrahydro-pyridine yields a mixture of (1R, 6R)-6-(4-benzyloxy-phenyl)-3-[(R)-1-phenyl-ethyl]-7-oxa-3-aza-bicyclo [4.1.0] heptane and of (1S, 6S)-6-(4-benzyloxy-phenyl)-3-[(R)-1-phenyl-ethyl]-7-oxa-3-aza-bicyclo [4.1.0] heptane by an epoxidation reaction.

Optionally the desired stereoisomer especially diastereomer can be isolated by methods known in the art such as crystallisation, chromatography or distillation, preferably crystallisation or chromatogaphy. These methods also include the formation of salts or derivatives of compounds of the formula 1 and in a following step the separation of these salts or derivatives by the above methods. These methods, especially methods for the separation of diastereoisomers are well known in the art and are for example described in Houben-Weyl, Methods of Organic Chemistry (pp. Vol. E21, p. 81, 91).

Preferred solvents taken alone or in combination which can be used for the crystallisation of compounds of formula 1 are protic or aprotic solvents which do not react with compounds of formula 1. Examples for such solvents are alcohols such as ethanol, isopropanol or methanol, esters such as ethyl acetate, ethers such as diethyl ether or diisopropyl ether, alone or in a suitable combination or also in combination with an appropriate amount of a hydrocarbon such as pentane or hexane. Particularly preferred is ethyl acetate and especially, wherein diethyl ether is added.

A preferred method of isolation of a diastereomer as above is crystallisation of a free compound of formula 1. Particularly the crystallisation of a free compound of formula 1 in ethyl acetate, by addition of diethyl ether.

After the formation of compounds of formula 1 the epoxide is opened by reaction with a metal alcoholate such as potassium t-butoxide, aluminium isopropoxide, titanium (IV) t-butoxide, with a lithium amide such as lithium diisopropylamide or with an organolithium compound such as phenyllithium, sec-butyllithium or methyllithium to give a compound of the general formula 5.

Moreover, a preferred aspect of the above process is the reaction of a compound of the formula 1 or a salt thereof, with phenyllithium. Particularly preferred is the above process, wherein the desired stereoisomer of a compound of the formula 1 reacts with phenyllithium. Solvents for this reaction taken alone or in combination are for example. ethers such as tetrahydrofuran, diethyl ether, or tert-butyl methyl ether, aromatic hydrocarbons such as toluene or chlorobenzene or pyridine. The solvent, which is preferred, depends on the reagent. In the case of phenyllithium as the reagent, tert-butyl methyl ether is a particularly preferred solvent.

The opening of the epoxide can be performed in a temperature range from about -40°C up to the boiling of the solvent. Preferred is a temperature range from about -25°C up to 0°C. Particularly preferred is a temperature of about -15°C.

The compound obtained can be used for the preparation of renin inhibitors as disclosed in WO 97/09311. In general, this preparation can be performed as follows: Alkylation of the 3-hydroxy function of for example (3S)-4-(4-benzyloxy-phenyl)-1-[(1R)-phenyl-ethyl]-1,2,3,6-tetrahydro-pyridin-3-ol can be performed in solvents as ethers like tetrahydrofuran and 1,2-dimethoxyethane, dimethylformamide or dimethylsulfoxide with aliphatic chlorides, bromides, iodides, tosylates or mesylates in the presence of a base like sodium hydride or potassium tert-butoxide. The alkylating agents used can either contain the whole substituent desired or optionally suitably protected functional groups which allow further structural modifications at a later stage of the synthesis. This substituent is represented by R' in formula 7 and 8.

Hydroboration of the ether compounds followed by subsequent basic oxidative working-up produces compounds of the general formula 7 with high diastereoselectivity. The hydroboration can be effected according to methods known per se, for example in a solvent which is inert under the reaction conditions, such as an ether, e.g. 1,2-dimethoxyethane, at a temperature between about 0°C and 70°C, and with a diborane-containing or diborane-liberating reagent such as e.g. borane in tetrahydrofuran or a mixture of sodium borohydride and boron trifluoride diethyletherate. The carboboranes which are formed as intermediates can be converted for example into the secondary alcohols of general formula 7 by reaction with bases, e.g. potassium hydroxide, and an oxidizing agent, e.g. hydrogen peroxide, at a temperature between about room temperature and 120°C. Reorganization of protective groups, removal of the R¹ and R² functions and re-introduction of a N-protective group by well established procedures as e.g.: Hydrogenolysis with hydrogen in the presence of a palladium catalyst followed by introduction of the Boc group with di-tert-butyldicarbonate in dioxane / water converts compounds of the general formula 7 into a compound of the following formula 8 bearing a phenolic and an aliphatic OH-function which an be functionalized selectively.

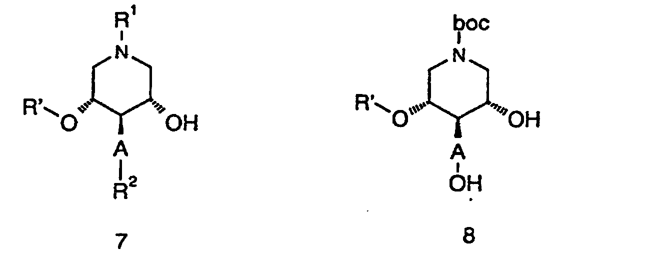

Selective functionalization of the phenolic function in compounds of the general formula 8 can be performed with alkylation reactions using aliphatic or benzylic chlorides, bromides, iodides, tosylates or mesylates in the presence of a base like potassium carbonate in solvents such as an ether like tetrahydrofuran, or in dimethylformamide, dimethylsulfoxide, acetone, methyl-ethyl-ketone, or pyridine at temperatures between 0°C and 140°C. The alkylating agents used can either contain the whole chain desired or optionally suitably protected functional groups which allow further structural modifications at a later stage of the synthesis. Functionalization at the secondary hydroxy function of the piperidine ring can then be performed in solvents as ethers like tetrahydrofuran or 1,2-dimethoxyethane, or in dimethylformamide or dimethylsulfoxide in the presence of a base like sodium hydride or potassium tert-butoxide and a suitable alkylating agent, preferentially an aryl methyl chloride, bromide, mesylate or tosylate at temperatures between 0°C and 40°C. The alkylating agents used can either contain the whole substituent desired or optionally suitably protected functional groups which allow further structural modifications at a later stage of the synthesis. Further structural variations can comprise removal of protective functions followed by functionalizations of the liberated functional groups, e.g. etherification of a phenolic moiety. Final removal of the Boc-protective group can be performed in the presence of acids such as hydrochloric, hydrobromic, sulfuric, phosphoric, trifluoroacetic acid in a variety of solvents such as alkohols and alcohol/water mixtures, ethers and chlorinated hydrocarbons. The Boc-protective group can also be removed with anhydrous zinc bromide in inert solvents such as dichloromethane.

The preparation of the starting compounds of formula 2 can be represented by the following scheme:

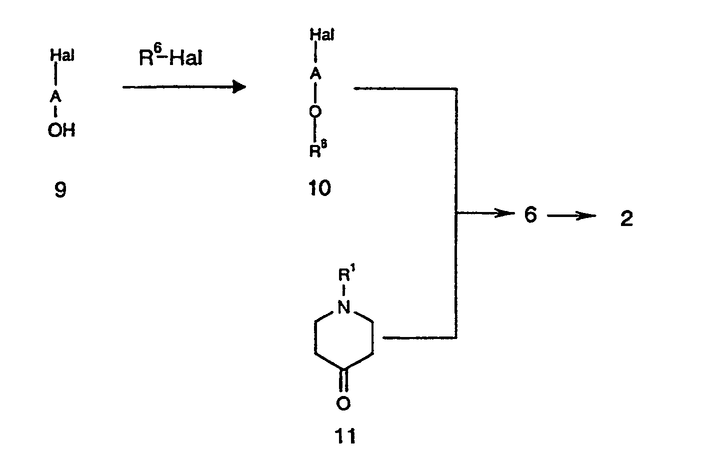

In detail, a compound of formula 2 can be obtained by reaction of compound 6 with an acid, e.g. oxalic acid dihydrate in an inert solvent, wherein compounds 6 are formed by reacting a compound of the formula 10 in an inert solvent with n-butyllithium or a Grignard reagent to form an organometallic intermediate which is reacted with a compound of the formula 11. The preparation of compound 10 can be performed by reacting a compound of the formula 9 with a compound of the formula R⁶-Hal in the presence of a base and preferably a catalyst such as NaI in an inert solvent.

R⁶ is alkyl, cycloalkyl, alkenyl, aryl, aralkyl, aralkoxyalkyl, alkylsulfonyl or arylsulfonyl. Compound 11 is obtainable for example by the reaction of R¹-NH₂ with 1-ethyl-1-methyl-4-oxo-piperidinium-iodide in the present of a base. 1-Ethyl-1-methyl-4-oxo-piperidinium-iodide is obtainable by the reaction of 1-ethyl-4-piperidone with methyl iodide in an inert solvent.
or

Alternatively, a compound of the formula 2 can be obtained by the reaction of an ammonium salt R¹-NH₃⁺X⁻ with formaldehyde and compound 3 which can be obtained by a reaction of methyltriphenylphosphoniumbromide, potassium tert-butoxide and compound 12 in an inert solvent. 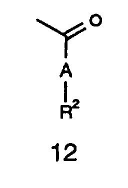 or

Alternatively, a compound 2 can be prepared by the reaction of an ammonium salt of the formula R¹-NH₃⁺X⁻ with formaldehyde and with a compound of the formula 4. Compound 4 is formed by the reaction of an organometallic compound containing a methyl group attached to the metal as in methylmagnesium bromide or methyllithium with compound 9, while compound 4, wherein R² means chlorine, bromine or iodine can be prepared via oxidation of a halocumene (for example described in US 3954876 or DE 2302751).

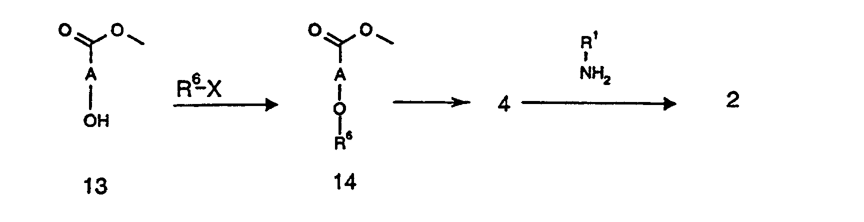

Alternatively compounds of formula 2 are obtainable by reacting a salt of the formula R¹-NH₃X with formaldehyde and compound 4. Preferably, R¹-NH₃X is generated in the reaction mixture from a compound R¹-NH₂ using the appropriate amount of a suitable acid HX. Furthermore, compound 4 can be obtained by the reaction of compound 14 with an adequate organometallic compound. Moreover, compound 14 is formed by the reaction of compound 13 with R⁶-X in the presence of a base in an inert solvent. R⁶ is defined as above.

Moreover, a compound of formula 1, wherein R² means chlorine, bromine or iodine is converted to the corresponding allylic alcohol of formula 5 by using a reagent such as aluminium isopropoxide. Furthermore, the halogen substituent can be replaced by e.g. an oxygen substituent, a carbon substituent or a nitrogen substituent by using an appropriate oxidant, by using conditions for transition metal catalyzed Heck or cross-coupling reactions or by using e.g. amination conditions with transition metal catalysis (S. L. Buchwald et al., J. Org. Chem. 1997, 62, 1568) respectively.

The following preparations and examples illustrate preferred embodiments of the present invention but are not intended to limit the scope of the invention.

### Examples

### Example 1

### (Preparation of product)

### Preparation of (1R, 6R)-6-(4-benzyloxy-phenyl)-3-[(R)-1-phenyl-ethyl]-7-oxa-3-aza-bicyclo [4.1.0] heptane

At room temperature 44.3 g of (R)-4-(4-Benzyloxy-phenyl)-1-(1-phenyl-ethyl)-1,2,3,6 tetrahydropyridine (120mmol) was suspended in 440mL of dioxane. Under stirring 40.4 g of 48% aqueous hydrogen bromide (240mmol) was added at 15-20 °C within 5 min, followed by 24mL of water. At the same temperature, the pH of the mixture was adjusted to pH 2 using 65mL of 2 N NaOH. The slightly turbid solution was cooled to 2-3°C, and 21.2 g of bromine (133mmol) was continuously added over 1.5h from a syringe pump via a teflon cannula. After the bromine addition, stirring at 2-3°C was continued for another 1.5 h. At this point, all starting material had reacted. At 0°C, 160mL of 4 N NaOH (640mmol) was added over 30 min and stirring was continued. After 2 h the intermediate had completely reacted to the final product. The reaction mixture was extracted using a mixture of 500mL of ethyl acetate and 200mL of 20% aq. sodium chloride. The aqueous phase was separated and extracted with 1 portion of 300mL of ethyl acetate. The organic phases were washed with 300 ml of 20 % aq. sodium chloride, combined, dried (MgSO₄) and evaporated under reduced pressure to give 51.3 g crude product as a brown oil. The crude product was taken up in 50mL of ethyl acetate. Immediately after dissolution crystals started to separate. 50mL of diethyl ether was added and the suspension was cooled to 0°C. A second portion of 25mL of diethyl ether was added and the suspension was stirred for another 1 h at 0 °C. The crystals were collected on a filter funnel and washed with a portion of cold diethyl ether, The product was then dried for 2 h at 26 mbar/45°C. There was obtained 23.5 g of (1R, 6R)-6-(4-benzyloxy-phenyl)-3-[(R)-1-phenyl-ethyl]-7-oxa-3-azabicyclo [4.1.0] heptane.

### Example 2

### (Preparation of the starting material)

### a) Preparation of 4-benzyloxybromobenzene

200 g (1.16mol) of 4-bromophenol was dissolved in 2.1 L of acetone under argon. Then 320 g (2.31mol) K₂CO₃ and 3.465 g (23.1mmol) NaI were added. The mixture was stirred at room temperature and 292.7 g (2.31mol) of benzyl chloride was added during 1h. Then the mixture was boiled during 48 h. The acetone (ca. 500mL) was partially removed on the rotary evaporator. 1.2 L 10 % aq. Na₂CO₃ was added to the residue. After extraction with ethyl acetate (1x1 L + 2x500mL) the organic phase was washed with 1 L of a half-saturated NaCl solution. After drying over Na₂SO₄ and concentrating, the main part of the benzyl chloride was removed. 400mL of pentane was added to the residue. The crystallisation began during stirring at 0°C. The crystals were separated and washed with 2x150mL pentane and dried during 2 h at 15 mbar (40C° bath temperature) and 2 h under high vacuum at room temperature. 230 g (75 %) 4-benzyloxybromobenzene was obtained.

### b) Preparation of 1-ethyl-1-methyl-4-oxo-piperidinium-iodide

To a solution of 93 g (730mmol) 1-ethyl-4-piperidone (Aldrich 27950-1) in 730mL acetone 124 g (876mmol) methyl iodide (Acros 12237) was added during 30 min. The temperature was kept at 25-30°C. The product began to precipitate after addition of 1/5 of the methyl iodide. The mixture was stirred for 5h at 22°C and 30 min at 0 °C. The cold suspension was filtered and the product was washed with acetone. 188 g (95 %) 1-ethyl-1-methyl-4-oxo-piperidinium iodide was obtained.

### c) Preparation of (R)-1-(1-phenyl-ethyl)-piperidin-4-one

a) Under argon 84.6 g (698mmol) (R)-(+)-1-phenylethylamine (Merck no. 807031) and 1.4 L ethanol were mixed. A solution of 203 g (1.47mol) K₂CO₃ in water was added. The mixture was heated at 80°C under stirring and a solution of 188 g (698mmol) 1-ethyl-1-methyl-4-oxo-piperidinium iodide in 700mL water was added during 1h. The mixture was heated again for 105 min under stirring and then ethanol was removed on the rotary evaporator.

The residue was extracted with dichloromethane (1x1.5 L + 1x1 L). The organic phases were washed with half-saturated NaCl solution (2x800mL) and dried with Na₂SO₄. After evaporation of the solvent 144 g crude (R)-1-(1-phenyl-ethyl)-piperidin-4-one was obtained. 70mL 37 % HCl were added at 5°C to 300mL of isopropanol during 30 min. The mixture was added during 2 h under stirring at 15-20°C to a solution of 144 g crude (R)-1-(1-phenyl-ethyl)-piperidin-4-one in 100mL ethylacetate. Crystallisation began after addition of 1/3 of the above mixture. The suspension was stirred overnight at room temperature and then for 3 h at 0°C. After adding 80mL of pentane the mixture was stirred again for 3 h at 0°C. The product was separated and washed with isopropanol (3x70mL). After drying the hydrochloride (188 g) was suspended in 1 L dichloromethane and 700mL of 10 % Na₂CO₃ was added. The organic phase was separated and washed with half-saturated NaCl (1x1L). After drying over MgSO₄ the organic phase was concentrated. The residue was dried during 2 h in high vacuum. 113 g (R)-1-(1-phenyl-ethyl)-piperidin-4-one was obtained.

### d) Preparation of (R)-4-(4-benzyloxy-phenyl)-1-(1-phenyl-ethyl)-piperidin-4-ol

Under argon 175.2 g (666mmol) 4-benzyloxybromobenzene was dissolved in 1.4 L dry THF (MS 4 A). The solution was cooled to -75°C and a solution of 416mL 1.6 M butyllithium (666mmol) in hexane was added during 40 min. After stirring for 1h a solution of 113 g (555mmol) (R)-1-(1-phenyl-ethyl)-piperidin-4-one in 400mL THF was added during 1h at -75°C. The mixture was stirred for another 1h and, after heating to room temperature, poured into 1.5 L of ice-water. The mixture was extracted with 1 L ethyl acetate. The organic phase was washed with 1 L of a half-saturated NaCl solution, dried over Na₂SO₄ and concentrated. 262 g of (R)-4-(4-benzyloxy-phenyl)-1-(1-phenyl-ethyl)-piperidin-4-ol was obtained.

### e) Preparation of (R)-4-(4-Benzyloxy-phenyl)-1-(1-phenyl-ethyl)-1,2,3,6 tetrahydropyridine

121.7 g crude (R)-4-(4-benzyloxy-phenyl)-1-(1-phenyl-ethyl)-piperidin-4-ol was dissolved at 40°C in 1.21 L dichloroethane. 59.4 g (471mmol) of oxalic acid dihydrate (Merck 492) was added. The mixture was boiled for 3 h, while 20mL of water was separated. The reaction mixture was washed at room temperature with 1.2 L 10 % Na₂CO₃. The precipitate (52 g) was separated from filtrate A and added to a mixture of 250mL 2 N NaOH and 300mL dichloromethane, where it was dissolved after stirring for 30 min at 30-35°C. The organic phase was separated and washed with a half-saturated NaCl solution. The obtained precipitate was separated and dissolved in 200mL dichloromethane and 60mL methanol. The combined organic phases were concentrated after drying over Na₂SO₄. 80mL ethyl acetate was added to the residue and stirred for 2 h. The crystals were separated, washed with pentane, and dried. 36.5 g of (R)-4-(4-benzyloxy-phenyl)-1-(1-phenyl-ethyl)-1,2,3,6-tetrahydropyridine was obtained.

The organic phase of the above-mentioned filtrate A was washed with 1.5 L of a half-saturated NaCl solution. After drying the organic phase was concentrated. 80mL ethylacetate and 30mL ether were added to the residue. After stirring for 3 h at 0°C the crystals were separated and then washed with ethyl acetate (2x20mL) and pentane (50mL) and dried. 33.0 g of (R)-4-(4-benzyloxy-phenyl)-1-(1-phenyl-ethyl)-1,2,3,6-tetrahydropyridine was obtained.

In total: 33.0 g + 36.5 g = 69.5 g (R)-4-(4-benzyloxy-phenyl)-1-(1-phenyl-ethyl)-1,2,3,6-tetrahydropyridine (73 % based on (R)-1-(1-phenyl-ethyl)-piperidin-4-on) was obtained.

### Example 3

### (Preparation of starting material)

### a) Preparation of 1-Isopropenyl-4-benzyloxy-benzene

At room temperature 29.6 g of methyltriphenylphosphonium bromide (83mmol) was suspended in 75mL of tetrahydrofuran. A solution of 9.2 g of potassium tert-butoxide (82mmol) in 35mL of tetrahydrofuran was added over 30 min, and the mixture was stirred for 10 min at room temperature and was then cooled to 0°C. At this temperature, a solution of 17.0 g of 4-benzyloxyacetophenone (75mmol) in 100mL of tetrahydrofuran was added during 1.5 h to the solution of the ylide. Stirring at 0°C was continued for 1 h, then 1mL of acetic acid was added to the reaction mixture. The reaction mixture was poured into a mixture of 300mL of saturated aq. sodium bicarbonate, 200 g of ice and 250mL of ethyl acetate. Then the aqueous phase was extracted with ethyl acetate. The organic phases were washed with 200mL of 20% aq. sodium chloride, combined, dried (Na₂SO₄) and evaporated under reduced pressure to give 40.5 g of a white solid residue. The residue was suspended in 250mL of hexane, and the mixture was stirred overnight at room temperature. The tripenylphosphinoxide was filtered off and washed with hexane. The filtrate was evaporated to give 15.8 g of a white solid. In order to remove traces of triphenylphosphine oxide, the product was passed through a pad of silica gel using hexane-ethyl acetate 95:5 (750mL) as eluent. The combined fractions containing the desired compound were evaporated. The residue was suspended in 80mL of pentane, then the product was collected by filtration, washed with pentane and dried to a constant weight. 14.1g 1-isopropenyl-4-benzyloxy-benzene was obtained.

### b) Preparation of (R)-4-(4-benzyloxy-phenyl)-1-(1-phenyl-ethyl)-1,2,3,6-tetrahydro-pyridine

At room temperature 20.7 g of (R)-1-phenylethylamine hydrochloride (131mmol) was dissolved in 60mL of water. 22mL of 36.5 % aqueous formaldehyde was added and the mixture was stirred 10 min at room temperature and then warmed up to 40 °C. At this temperature, a solution of 26.75 g of 1-isopropenyl-4-benzyloxy-benzene (119mmol) in a mixture of 30mL of dioxane and 74mL of dichloromethane was continuously added over 1.25 h. During and after the addition of the olefin solution, dichloromethane was distilled off. After the removal of dichloromethane, the reaction mixture was stirred at 70°C overnight. A solution of 9.96 g of conc. sulphuric acid (99mmol) in 30mL of water was added during 5 min to the reaction mixture which was then heated to 95-100°C and stirred at this temperature for 5.5 h. The reaction mixture was slowly poured into a mixture of 250mL of 10 % aq. sodium carbonate and ice and then extracted with 600mL of dichloromethane. The organic phases were extracted with one portion of 600mL of 20 % aq. sodium chloride, combined, dried (Na₂SO₄) and evaporated under reduced pressure to give 64 g crude product as a brown-red oil which partially crystallised. The crude product was dissolved in 250mL of dichloromethane. 120mL of isopropanol was added and the dichloromethane as well as a small part of the isopropanol was distilled off at reduced pressure (rotary evaporator, bath 45°C). White crystals started to precipitate, and the suspension was stirred at 0°C for 2 h. The crystals were collected on a filter funnel and washed with three portions of 50mL of cold isopropanol and with 60mL of hexane. 29.2 g (66%) (R)-4-(4-benzyloxyphenyl)-1-(1-phenyl-ethyl)-1,2,3,6-tetrahydro-pyridine was obtained after drying for 2 h at 16 mbar/50°C and for 2 h at 0.2 mbar/22°C.

### Example 4

### (Preparation of starting material)

### a) Preparation of 2-(4-benzyloxy-phenyl)-propan-2-ol

The reaction flask was charged under argon with 3.45 g of magnesium (142mmol). A solution of 21.16 g of methyl iodide (147mmol) in 120mL of tert-butyl-methyl-ether was added during 45 min at 45 °C under stirring. Then stirring was continued for 1 h at 45 °C and then a solution of 27.12 g of 4-benzyloxyacetophenone (120mmol) in 100mL of tetrahydrofuran was added during 45 min., while a temperature of 45°C was again maintained. Stirring at 45 °C was continued for 1.5 h. After cooling to room temperature, the white suspension was poured into a mixture of 100mL of 10% aqueous ammonium chloride and of ice and extracted with 150mL of ethyl acetate. The aqueous phase was separated and extracted with 100mL of ethyl acetate. The organic phase was washed with 120mL of 20% aq. sodium chloride, combined, dried (MgSO₄) and evaporated under reduced pressure to give 29.9 g of crude product as an oil which partially crystallised. The crude product was taken up in 30mL of dichloromethane. The solution was concentrated at the rotary evaporator almost to dryness. Then 6mL of ethyl acetate was added followed by gradual addition of a total of 180mL of hexane. The suspension was then kept at 0°C for 30 min. The crystals were collected and washed with cold hexane. After drying for 2 h at 16 mbar/45°C 26.7 g (92%) 2-(4-benzyloxy-phenyl)-propan-2-ol was obtained.

### b) Preparation of (R)-4-(4-benzyloxy-phenyl)-1-(1-phenyl-ethyl)-1,2,3,6-tetrahydro-pyridine

At room temperature 6.94 g of (R)-1-phenylethylamine hydrochloride (44mmol) was dissolved in 24mL of water. 8.0 g of 36.5 % aqueous formaldehyde (2.92 g HCHO, 97mmol) was added and the mixture was stirred for 10 min. Then, a solution of 9.68 g of 2-(4-benzyloxy-phenyl)-propan-2-ol (40mmol) in 10mL of dioxane was added. The reaction mixture was heated to 70°C and stirred overnight at this temperature. A solution of 1.72 g of conc. sulphuric acid (17.6mmol) in 8mL of water was added to the reaction mixture within 5 min. Then the mixture was heated to 100 °C and stirred at this temperature for 7 h. The reaction mixture was slowly poured into a mixture of 150mL of 10% aq. sodium carbonate and 50 g of ice and extracted with 450mL of dichloromethane. The organic phases were extracted with 150mL of water, combined, dried (Na₂SO₄) and evaporated under reduced pressure to give 18.1 g crude product as a orange-red oil which partially crystallised.

The crude product was dissolved in 60mL of dichloromethane. 80 mL of isopropanol was added and the dichloromethane as well as a small part of the isopropanol was distilled off at 400 mbar (rotary evaporator, bath 55°C). White crystals precipitated, and the suspension was stirred 1 h at room temperature and additionally 1 h at 5°C. The crystals were collected and washed with 2 portions of 25mL isopropanol and with 2 portions of 25 mL hexane. The product was then dried for 2 h at 16 mbar/40°C and for 3 h at 0.2 mbar/22°C. 9.1 g (61%) of (R)-4-(4-benzyloxy-phenyl)-1-(1-phenylethyl)-1,2,3,6-tetrahydro-pyridine was obtained.

### c) Preparation of (R)-1-phenylethylamin hydrochloride:

At room temperature 122 g of (R)-1-phenylethylamine (1.0mol) was dissolved in 30mL of isopropanol. The solution was stirred and cooled to 0°C. Then, a previously prepared solution of 100mL of 37 % hydrochloric acid (118 g, 1.2mol) in 320mL of isopropanol was added during 1 h. The solution was stirred at 0 °C for an additional 40 min, and then it was concentrated on a rotary evaporator (16 mbar, bath 45°C) to a volume of 300mL. The translucent gel which had formed was transferred into a 1.5 l flask, then, under stirring, 250mL of tert-butyl-methyl-ether was slowly added. Crystals started to form and the suspension was stirred at 0°C for 3 h. The product was collected by filtration, washed with 100mL of tert-butyl-methyl-ether and dried at 30°C/16 mbar for 4 hours. 133 g (84 %) of 1-phenylethylamine hydrochloride was obtained.

### Example 5

### (Preparation of starting material)

### a) Preparation of methyl 4-benzyloxybenzoate

To a solution of 15.2 g of methyl-4-hydroxybenzoate (100mmol) in 125mL of N,N-dimethylformamide was added under stirring 33.13 g of potassium carbonate (240mmol). Then 17.45 g of benzyl bromide (102mmol) was added within 5 min. The mixture was stirred at 25°C using a water bath. The reaction was complete after 3h. The reaction mixture was poured into a mixture of 180 g of ice and 200mL of ethyl acetate. After extraction, the aqueous phase was separated and extracted with three portions of 80 mL of ethyl acetate. The organic phase was washed with two portions of 150mL of water, combined, dried (MgSO₄) and partially concentrated to give a thick suspension. 60mL of pentane was added and the suspension was stirred during 2 h at room temperature. The crystalline methyl 4-benzyloxybenzoate was collected on a filter, washed with pentane and dried.

### b) Preparation of 2-(4-benzyloxy-phenyl-propan-2-ol

Under argon 6.63 g of magnesium (273mmol) was suspended in 15mL of tert-butyl methyl ether. A solution of 38.68 g of methyl iodide (273mmol) in 145mL of tert-butyl methyl ether was added during 45 min under stirring while maintaining the temperature at 40°C. Then stirring was continued at 40°C for 1.5 h and then the mixture was cooled to room temperature. A solution of 30.0 g of methyl 4-benzyloxybenzoate (124 mmol) in 120mL of tetrahydrofuran was then added during 1 h. The temperature was kept at 20°C. After complete addition, the reaction mixture was heated to 42°C and stirred 3 h at this temperature. After cooling to room temperature, the reaction mixture was poured into a mixture of 300mL of 10 % aqueous ammonium chloride and 100 g of ice and extracted with ethyl acetate. The organic phases were washed with water and saturated aqueous sodium bicarbonate, combined, dried and evaporated to give the crude product as an oil which partially crystallised. The product was dissolved at 25°C in diethyl ether. When crystals started to separate the solution was cooled to 18°C. After 30min hexane was added. The suspension was then stirred for 1h at 5°C. The crystalline 2-(4-benzyloxy-phenyl)-propan-2-ol was collected on a filter and washed with hexane.

### c) Preparation of (R)-4-(4-benzyloxy-phenyl)-1-(1-phenyl-ethyl)-1,2,3,6-tetrahydro-pyridine

At room temperature, the reaction flask was charged with 10.66 g of (R)-1-phenylethylamine (88mmol) and 40mL of water. Under stirring, the pH of the mixture was adjusted to a value of 4.1 by slow addition of aqueous hydrochloric acid. Then 16.0 g of 36.5 % aqueous formaldehyde (5.84 g HCHO, 194mmol) was added and the mixture was stirred for 10 min. A solution of 19.38 g of 2-(4-benzyloxy-phenyl)-propan-2-ol (80mmol in 20mL of dioxane) was then added. The reaction mixture was heated to 70°C and stirred overnight at this temperature. A solution of 3.44 g of conc. sulphuric acid (35mmol) in 16mL of water was added during 5 min to the reaction mixture which was then heated to 100°C and stirred at this temperature for 7 h. The reaction mixture was slowly poured into a mixture of 300mL of 10% aq. sodium carbonate and 100 g of ice and extracted with dichloromethane. The organic phases were extracted with water, combined, dried and evaporated to an orange-red oil which partially crystallised. The crude product was dissolved in 120mL of dichloromethane. 160mL of isopropanol was added and the dichloromethane as well as a part of the isopropanol was distilled off at 400 mbar (rotary evaporator, bath 55 °C). White crystals precipitated. The crystals were collected on a filter funnel and washed with isopropanol and then with hexane. The obtained (R)-4-(4-benzyloxy-phenyl)-1-(1-phenyl-ethyl)-1,2,3,6-tetrahydro-pyridine was then dried for 2 h at 16 mbar/40°C and for 3 h at 0.2 mbar/22°C.

### Example 6

### (Preparation of a precursor for renin inhibitors)

### Preparation of (3S)-4-(4-benzyloxy-phenyl)-1-[(1R)-phenyl-ethyl]-1,2,3,6-tetrahydro-pyridin-3-ol

Under argon 5.77 g of (1R, 6R)-6-(4-benzyloxy-phenyl)-3-[(R)-1-phenyl-ethyl]-7-oxa-3-aza-bicyclo [4.1.0] heptane (15mmol) and 225mL of tert-butyl methyl ether were charged in the reaction vessel. The mixture was cooled under stirring to -15°C and, at this temperature, 18.75mL of 1.6M phenyllithium (30mmol) was continuously added over 45min from a syringe pump via a teflon cannula. Stirring was continued for a 3.5 h. At this point the reaction was complete. The light brown reaction mixture was poured onto a mixture of 100mL of 7 % aqueous sodium bicarbonate and of ice and extracted with 250mL of ethyl acetate. The aqueous phase was separated and extracted with a fresh portion of 250ml of ethyl acetate. The organic phases were washed with two portions of 100mL i.e. 200mL of 20 % aq. sodium chloride, combined, dried (MgSO₄) and evaporated under reduced pressure to give 6.8 g crude product as a brown solid. The crude product was taken up in 30mL of dichloromethane. The solution was concentrated at the rotary evaporator to an oil of approx. 9 g weight. 10mL of ethyl acetate was added and crystals started to separate. A second portion of 5mL of ethyl acetate was added followed by approx. 25mL of hexane. The suspension was then stirred at 0°C for 2 h. The crystals were collected and washed with a portion of cold hexane. The product was dried for 2 h at 16 mbar/45°C. There was obtained 5.0 g (86%) of(3S)-4-(4-benzyloxy-phenyl)-1-[(1R)-phenyl-ethyl]-1,2,3,6-tetrahydro-pyridin-3-ol as light brown crystals, m.p. 112-114°C.

### Example 7

### (Preparation of renin inhibitor)

### Preparation of (3R,4S,5S)-4-[4-(3-benzyloxy-propoxy)-phenyl]-5-ethoxy-3-(naphthalen-2-ylmethoxy)-piperidine

a) 49.3 g (128mmol) of (3S)-4-(4-benzyloxy-phenyl)-1-[(1R)-phenyl-ethyl]-1,2,3,6-tetrahydro-pyridin-3-ol were dissolved in 250 ml of N,N-dimethylformamide, treated portionwise with 25 g (about 600mmol) of sodium hydride dispersion in refined oil (55-65%) and the reaction mixture was heated to 50°C under argon for 1 hour. After cooling to 5°C the mixture was treated slowly with 23 ml (285mmol) of ethyl iodide and stirred without cooling for one hour. Thereupon, the reaction mixture was poured into 2 litres of ice-water and extracted three times with 1 litre of ethyl acetate. The combined ethyl acetate phases were subsequently washed with water, dried over magnesium sulfate and evaporated on a rotary evaporator at a maximum 40°C. The residue which was thereby obtained was chromatographed on silica gel with hexane/ethyl acetate. There were thus obtained (3S)-4-(4-benzyloxy-phenyl)-3-ethoxy-1-[(1R)-phenyl-ethyl]-1,2,3,6-tetrahydro-pyridine as a colourless oil.
b) 35 g (84.6mmol) of (3S)-4-(4-benzyloxy-phenyl)-3-ethoxy-1-[(1R)-phenyl-ethyl]-1,2,3,6-tetrahydro-pyridine were dissolved in 500 ml of 1,2-dimethoxyethane, treated with 9.91 g (262mmol) of sodium borohydride and then treated while cooling at a maximum 28°C with a solution of 44.3 ml (353mmol) of boron trifluoride etherate in 44.3ml of 1,2-dimethoxyethane and the reaction mixture was stirred at room temperature for 2 hours. Subsequently, while cooling at a maximum of 35°C, 169 ml of 4.1N potassium hydroxide solution followed by 33.9 ml of 30% hydrogen peroxide solution were added dropwise and the reaction mixture was heated under reflux for 3 hours. After cooling to room temperature the reaction solution was poured into 2 liters of water and extracted twice with 1 litre of dichloromethane each time. The combined dichloromethane phases were washed with water, dried over magnesium sulphate and evaporated on a rotary evaporator at a maximum 40°C. The residue which was thereby obtained was chromatographed on silica gel with hexane/ ethyl acetate. There were thus obtained (3R,4R,5S)-(4-benzyloxy-phenyl)-5-ethoxy-1-[(1R)-phenyl-ethyl]-piperidin-3-ol) as a colourless oil.
c) 20 g (46.3mmol) (3R,4R,5S)-(4-benzyloxy-phenyl)-5-ethoxy-1-[(1R)-phenyl-ethyl]-piperidin-3-ol dissolved in 500 ml methanol were hydrogenated in the presence of 3.5 g of palladium catalyst (10% on charcoal) for 7 hours. The reaction mixture was then filtered and evaporated yielding crude (3R,4R,5S)-5-ethoxy-4-(4-hydroxy-phenyl)-piperidin-3-ol MS: 237(M⁺).
d) 11 g (46.3mmol) crude (3R,4R,5S)-5-ethoxy-4-(4-hydroxy-phenyl)-piperidin-3-ol were dissolved in 100 ml dioxane/50 ml water and treated with 11 g (50mmol) di-tert-butyldicarbonate and 8.4 g (100mmol) sodium hydrogencarbonate. The reaction mixture was then stirred for 2 hours. 100 ml 2 N NaOH were then added and the mixture again stirred for an additional hour. It was then acidified with solid citric acid. Then, the product was extracted 3 times with dichloromethane, the organic phases were washed twice with distilled water, then dried over magnesium sulphate, filtered and concentrated in a water-jet vacuum. The thus-obtained crude product was chromatographed on silica gel with dichloromethane/ethyl acetate. There were thus obtained (3R,4R,5S)-5-ethoxy-3-hydroxy-4-(4-hydroxy-phenyl)-piperidine-1-carboxylic acid tert-butylester as colourless oil; MS: 338 (M+H⁺).
e) A solution of 11.8 g (35.0mmol) of (3R,4R,5S)-5-ethoxy-3-hydroxy-4-(4-hydroxy-phenyl)-piperidine-1-carboxylic acid tert-butylester in 40 ml of dimethylformamide was treated in succession with 10.3 g (45.0mmol) of 3-bromo-propoxymethyl-benzene and 8.29 g (60.0mmol) of potassium carbonate. This mixture was stirred at 120°C for 26 hours. Subsequently, it was filtered, concentrated to a few millilitres, poured into 300 ml of an ice/water mixture and extracted three times with 100 ml of dichloromethane each time. The combined organic phases were washed once with a small amount of water, dried over magnesium sulphate, evaporated under reduced pressure and dried in a high vacuum. The thus-obtained crude product was separated on silica gel using a mixture of dichloromethane and methanol as the eluent and yielded (3R,4R,5S)-4-[4-(3-benzyloxy-propoxy)-phenyl]-5-ethoxy-3-hydroxy-piperidine-1-carboxylic acid tert-butylester as colourless oil; MS: 486 (M+H⁺); 508 (M+Na⁺).
f) 14.6 g (30.0mmol) of (3R,4R,5S)-4-[4-(3-benzyloxy-propoxy)-phenyl]-5-ethoxy-3-hydroxy-piperidine-1-carboxylic acid tert-butylester and 7.74 g (35.0mmol) of 2-bromomethyl-naphthalene were dissolved in 110 ml of dimethylformamide under argon and then 1.77 g (40.0mmol) of sodium hydride dispersion (55% in mineral oil) was added. Subsequently, the mixture was stirred at room temperature for 5 hours. The reaction mixture was poured onto ice-water, the product was extracted 3 times with dichloromethane, the organic phases were washed twice with distilled water, then dried over magnesium sulfate, filtered and concentrated in a water-jet vacuum. The thus-obtained crude product was chromatographed on silica gel with dichloromethane and methanol. (3R,4S,5S)-4-[4-(3-benzyloxy-propoxy)-phenyl]-5-ethoxy-3-(naphthalen-2-ylmethoxy)-piperidine-1-carboxylic acid tert-butylester was obtained as colourless oil; MS: 626.5(M+H⁺).
g) 14.4 g (23.0mmol) of (3R,4S,5S)-4-[4-(3-benzyloxy-propoxy)-phenyl]-5-ethoxy-3-(naphthalen-2-ylmethoxy)-piperidine-1-carboxylic acid tert-butylester were placed in 350 ml of abs. methanol at 0°C, then 24 ml (48mmol) of hydrochloric acid in methanol (2.0 molar) were added dropwise at 5°C max. and thereafter the mixture was warmed to room temperature. After 120 minutes the reaction mixture was poured into ice-cold sodium hydrogen carbonate solution and the product was extracted three times with dichloromethane, the organic phases were washed once with distilled water, then dried over magnesium sulphate, filtered and concentrated in a water-jet vacuum. The thus-obtained crude product was chromatographed on silica gel with dichloromethane and methanol. There were thus obtained (3R,4S,5S)-4-[4-(3-benzyloxy-propoxy)-phenyl]-5-ethoxy-3-(naphthalen-2-ylmethoxy)-piperidine as colourless oil; MS: 526(M+H⁺).

## Claims

1. A process for the preparation of a compound of formula 1 or a salt thereof 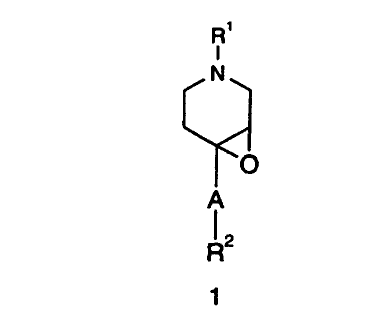 **characterised in that** the process comprises
a) epoxidation of a compound of formula 2 or a salt thereof 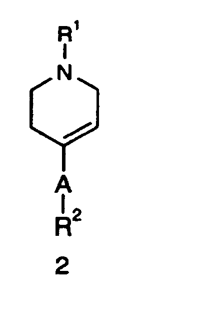 in which formulae
A is arylene;
R¹ is -C*HR⁴R⁵;
R² is -O-alkyl, -O-cycloalkyl, -O-alkenyl, -O-aryl, -O-aralkyl, -O-aralkoxyalkyl, -O-alkylsulfonyl, -O-arylsulfonyl, chlorine, bromine or iodine;
R⁴ is aryl;
R⁵ is alkyl, cycloalkyl, aryl, alkoxyalkyl or hydroxyalkyl;
and, wherein C* is an asymmetric carbon atom;
b) optionally followed by isolation of the desired stereoisomer;
wherein the term "alkyl" means alone or in combination a branched or unbranched alkyl group containing 1 to 8 carbon atoms; and
the term "aryl" means alone or in combination a phenyl or a naphthyl group which can be substituted by one or several substituents chosen from alkyl, cycloalkyl, alkoxy, halogen, carboxy, alkoxycarbonyl, hydroxy, amino, nitro, trifluoromethyl
the term "cycloalkyl" means alone or in combination a cycloalkyl cycle with 3 to 8 carbon atoms and preferred a cycloalkyl cycle with 3 to 6 carbon atoms;
the term "alkenyl" means alkenyl groups of 2 to 8 carbon atoms;
the term "arylene" means alone or in combination a phenylene or a naphthylene group which can be additionally substituted by one or several substituents chosen from alkyl, cycloalkyl, halogen, nitro, alkoxy, hydroxy, amino.

2. The process according to claim 1, wherein R⁵ is alkyl or cycloalkyl.

3. The process according to claim 1 or 2, wherein R⁴ is phenyl which is optionally substituted by one or more groups independently selected from alkyl, halogen or nitro.

4. The process according to any of claims 1 to 3, wherein R⁴ is phenyl and R⁶ is methyl.

5. The process according to any of claims 1 to 4, wherein A is phenylene and, wherein phenylene optionally is substituted by one to four additional substituents independently selected from alkyl, halogen or nitro.

6. The process according to any of claims 1 to 5, wherein R² is -O-benzyl or -O-methyl

7. The process according to any of claims 1 to 6, wherein a compound of formula 2 is reacted with a halogen or a halogen delivering agent.

8. The process of claim 7, wherein bromine is used.

9. The process according to any of claims 1 to 8, wherein the desired stereoisomer of a compound of formula 1 or a salt thereof is isolated by crystallisation.

10. The process according to any of claims 1 to 9 followed by a reaction with a metal alcoholate, a lithium amide or an organolithium compound to give a compound of the general formula 5 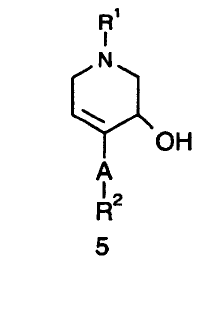 or a salt thereof.

11. The process according to any of claims 1 to 10, wherein a compound of formula 1 or a salt thereof is converted to (3R,4S,5S)-4-[4-(3-benzyloxy-propoxy)-phenyl]-5-ethoxy-3-(naphthalen-2-ylmethoxy)-piperidine **characterised in that**
a) (1R, 6R)-6-(4-benzyloxy-phenyl)-3-[(R)-1-phenyl-ethyl]-7-oxa-3-aza-bicyclo [4.1.0] heptane is transformed into (3S)-4-(4-benzyloxy-phenyl)-1-[(1R)-phenyl-ethyl]-1,2,3,6-tetrahydro-pyridin-3-ol by reaction with phenyllithium;
b) reaction with sodium hydride and ethyl iodide yields (3S)-4-(4-benzyloxy-phenyl)-3-ethoxy-1-[(1R)-phenyl-ethyl]-1,2,3,6-tetrahydropyridine;
c) hydroboration with sodium borohydride and boron trifluoride etherate followed by reaction with potassium hydroxide and hydrogen peroxide yields (3R,4R,5S)-(4-benzyloxy-phenyl)-5-ethoxy-1-[(1R)-phenyl-ethyl]-piperidin-3-ol);
d) hydrdrogenolysis yields (3R,4R,5S)-5-ethoxy-4-(4-hydroxy-phenyl)-piperidin-3-ol;
e) reaction with di-tert.-butyldicarbonate and sodium hydrogencarbonate followed by addition of NaOH yields (3R,4R,5S)-5-ethoxy-3-hydroxy-4-(4-hydroxy-phenyl)-piperidine-1-carboxylic acid tert-butylester;
f) treatment with 3-bromo-propoxymethyl-benzene and potassium carbonate yields (3R,4R,5S)-4-[4-(3-benzyloxy-propoxy)-phenyl]-5-ethoxy-3-hydroxy-piperidine-1-carboxylic acid tert-butylester;
g) reaction with 2-bromomethyl-naphthalene and sodium hydride yields (3R,4S,5S)-4-[4-(3-benzyloxy-propoxy)-phenyl]-5-ethoxy-3-(naphthalen-2-ylmethoxy)-piperidine-1-carboxylic acid tert-butylester;
h) reaction with hydrochloric acid yields (3R,4S,5S)-4-[4-(3-benzyloxypropoxy)-phenyl]-5-ethoxy-3-(naphthalen-2-ylmethoxy)-piperidine.

12. A compound according to formula 1 or a salt thereof, wherein R¹, R², R⁴, R⁵ and A are defined as in any of claims 1 to 6.

13. A compound according to formula 2 or a salt thereof, wherein R¹, R², R⁴, R⁵ and A are defined as in any of claims 1 to 6.

14. A compound according to formula 5 or a salt thereof, wherein R¹, R², R⁴, R⁶ and A are defined as in any of claims 1 to 6.

15. A compound according to any of claims 12, 13 and 14 selected from:
(1R, 6R)-6-(4-benzyloxy-phenyl)-3-[(R)-1-phenyl-ethyl]-7-oxa-3-aza-bicyclo [4.1.0] heptane;
(R)-4-(4-benzyloxy-phenyl)-1-(1-phenyl-ethyl)-1,2,3,6-tetrahydro-pyridine;
(R)-4-(4-benzyloxy-phenyl)-1-(1-phenyl-ethyl)-piperidin-4-ol;
(3S)-4-(4-benzyloxy-phenyl)-1-[(1R)-phenyl-ethyl]-1,2,3,6-tetrahydropyridin-3-ol.

16. The use of a compound according to claim 12 in the preparation of renin inhibitors.

17. A compound as obtained by the process according to any of claims 1 to 10.

## Patentansprüche

1. Verfahren zur Herstellung einer Verbindung der Formel 1 oder eines Salzes davon 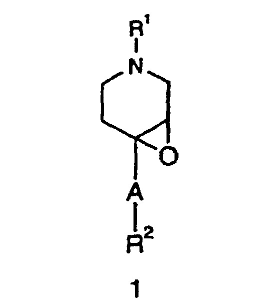 **dadurch gekennzeichnet, daß** das Verfahren umfaßt
a) Epoxidieren einer Verbindung der Formel 2 oder eines Salzes davon 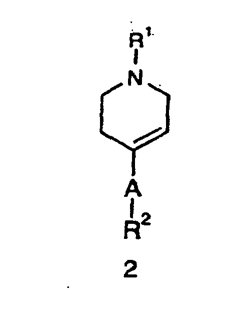 wobei in den Formeln
A Arylen ist;
R¹ -C*HR⁴R⁵ ist;
R² -O-Alkyl, -O-Cycloalkyl, -O-Alkenyl, -O-Aryl, -O-Aralkyl, -O-Arakoxyalkyl, -O-Alkylsulfonyl, -O-Arylsulfonyl, Chlor, Brom oder Iod ist;
R⁴ Aryl ist;
R⁵ Alkyl, Cycloalkyl, Aryl, Alkoxyalkyl oder Hydroxyalkyl ist;
und wobei C* ein asymmetrisches Kohlenstoffatom ist;
b) gegebenenfalls gefolgt von Isolieren des gewünschten Stereoisomers;
wobei der Ausdruck "Alkyl" allein oder in Kombination einen verzweigten oder unverzweigten Alkylrest mit 1 bis 8 Kohlenstoffatomen bedeutet;
der Ausdruck "Aryl" allein oder in Kombination einen Phenyl- oder Naphthylrest bedeutet, der mit einem oder mehreren Substituenten, ausgewählt aus Alkyl, Cycloalkyl, Alkoxy, Halogen, Carboxy, Alkoxycarbonyl, Hydroxy, Amino, Nitro, Trifluormethyl, substituiert sein kann;
der Ausdruck "Cycloalkyl" allein oder in Kombination einen Cycloalkylring mit 3 bis 8 Kohlenstoffatomen und bevorzugt einen Cycloalkylring mit 3 bis 6 Kohlenstoffatomen bedeutet;
der Ausdruck "Alkenyl" Alkenylreste mit 2 bis 8 Kohlenstoffatomen bedeutet; und
der Ausdruck "Arylen" allein oder in Kombination einen Phenylen- oder Naphthylenrest bedeutet, der zusätzlich mit einem oder mehreren Substituenten, ausgewählt aus Alkyl, Cycloalkyl, Halogen, Nitro, Alkoxy, Hydroxy, Amino, substituiert sein kann.

2. Verfahren nach Anspruch 1, wobei R⁵ Alkyl oder Cycloalkyl ist.

3. Verfahren nach Anspruch 1 oder 2, wobei R⁴ Phenyl, gegebenenfalls substituiert mit einem oder mehreren Resten, die unabhängig aus Alkyl, Halogen oder Nitro ausgewählt sind, ist.

4. Verfahren nach einem der Ansprüche 1 bis 3, wobei R⁴ Phenyl ist und R⁵ Methyl ist.

5. Verfahren nach einem der Ansprüche 1 bis 4, wobei A Phenylen ist und wobei Phenylen gegebenenfalls mit ein bis vier zusätzlichen Substituenten, unabhängig ausgewählt aus Alkyl, Halogen oder Nitro, substituiert ist.

6. Verfahren nach einem der Ansprüche 1 bis 5, wobei R² -O-Benzyl oder -O-Methyl ist.

7. Verfahren nach einem der Ansprüche 1 bis 6, wobei eine Verbindung der Formel 2 mit einem Halogen oder einem Halogen abgebenden Mittel umgesetzt wird.

8. Verfahren nach Anspruch 7, wobei Brom verwendet wird.

9. Verfahren nach einem der Ansprüche 1 bis 8, wobei das gewünschte Stereoisomer einer Verbindung der Formel 1 oder eines Salzes davon durch Kristallisation isoliert wird.

10. Verfahren nach einem der Ansprüche 1 bis 9, gefolgt von einer Umsetzung mit einem Metallalkoholat, einem Lithiumamid oder einer Organolithiumverbindung, um eine Verbindung der allgemeinen Formel 5 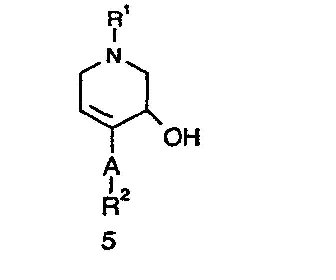 oder ein Salz davon zu ergeben.

11. Verfahren nach einem der Ansprüche 1 bis 10, wobei eine Verbindung der Formel 1 oder ein Salz davon in (3R,4S,5S)-4-[4-(3-Benzyloxy-propoxy)-phenyl]-5-ethoxy-3-(naphthalin-2-ylmethoxy)piperidin umgewandelt wird, **dadurch gekennzeichnet, daß**
a) (1R,6R)-6-(4-Benzyloxy-phenyl)-3-[(R)-1-phenyl-ethyl]-7-oxa-3-azabicyclo[4.1.0]heptan durch Umsetzung mit Phenyllithium in (3S)-4-(4-Benzyloxy-phenyl)-1-[(R)-phenyl-ethyl]-1,2,3,5-tetrahydropyridin-3-ol umgewandelt wird;
b) eine Umsetzung mit Natriumhydrid und Ethyliodid (3S)-4-(4-Benzyloxyphenyl)-3-ethoxy-1-[(1R)-phenyl-ethyl]-1,2,3,6-tetrahydropyridin liefert;
c) eine Hydroborierung mit Natriumborhydrid und Bortrifluoridetherat gefolgt von Umsetzung mit Kaliumhydroxid und Wasserstoffperoxid (3R,4R,5S)-(4-Benzyloxy-phenyl)-5-ethoxy-1-[(1R)-phenyl-ethyl]-piperidm-3-ol) liefert;
d) eine Hydrogenolyse (3R,4R,5S)-5-Ethoxy-4-(4-hydroxy-phenyl)-piperidin-3-ol liefert;
e) eine Umsetzung mit Di-tert.-butyldicarbonat und Natriumhydrogencarbonat gefolgt von Zugabe von NaOH (3R,4R,5S)-5-Ethoxy-3-hydroxy-4-(4-hydroxyphenyl)-piperidin-1-carbonsäure-tert.-butylester liefert;
f) eine Behandlung mit 3-Brom-propoxymethyl-benzol und Kaliumcarbonat (3R,4R,5S)-4-[4-(3-Benzyloxy-propoxy)-phenyl]-5-ethoxy-3-hydroxy-piperidin-1-carbonsäure-tert.-butylester liefert;
g) eine Umsetzung mit 2-Brommethyl-naphthalin und Natriumhydrid (3R,4S,5S)-4-[4-(3-Benzyloxy-propoxy)-phenyl]-5-ethoxy-3-(naphthalin-2-ylmethoxy)-piperidin-1-carbonsäure-tert.-butylester liefert;
h) eine Umsetzung mit Chlorwasserstoffsäure (3R,4S,5S)-4-[4-(3-Benzyloxypropoxy)-phenyl]-5-ethoxy-3-(naphthalin-2-ylmethoxy)-piperidin liefert.

12. Verbindung gemäß Formel 1 oder ein Salz davon, wobei R¹, R², R⁴, R⁵ und A wie in einem der Ansprüche 1 bis 6 definiert sind.

13. Verbindung gemäß Formel 2 oder ein Salz davon, wobei R¹, R², R⁴, R⁵ und A wie in einem der Ansprüche 1 bis 6 definiert sind.

14. Verbindung gemäß Formel 5 oder ein Salz davon, wobei R¹, R², R⁴, R⁵ und A wie in einem der Ansprüche 1 bis 6 definiert sind.

15. Verbindung nach einem der Ansprüche 12, 13 und 14, ausgewählt aus:
(1R,6R)-6-(4-Benzyloxy-phenyl)-3-[(R)-1-phenyl-ethyl]-7-oxa-3-azabicyclo[4.1.0]heptan;
(R)-4-(4-Benzyloxy-phenyl)-1-(1-phenyl-ethyl)-1,2,3,6-tetrahydropyridin;
(R)-4-(4-Benzyloxy-phenyl)-1-(1-phenyl-ethyl)-piperidin-4-ol;
(3S)-4-(4-Benzyloxy-phenyl)-1-[(1R)-phenyl-ethyl]-1,2,3,6-tetrahydropyridin-3-ol.

16. Verwendung einer Verbindung nach Anspruch 12 bei der Herstellung von Renininhibitoren.

17. Verbindung, erhalten durch das Verfahren nach einem der Ansprüche 1 bis 10.

## Revendications

1. Procédé de préparation d'un composé de formule 1 ou d'un sel de celui-ci 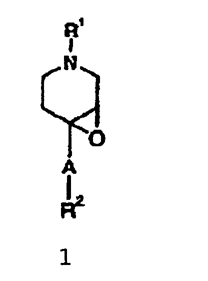 **caractérisé en ce que** le procédé comprend :
a) une époxydation d'un composé de formule 2 ou d'un sel de celui-ci 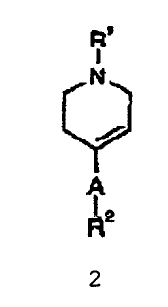 dans lesquelles :
A représente un groupe arylène ;
R¹ représente -C*HR⁴R⁵ ;
R² représente un groupe -O-alkyle, un groupe -O- cycloalkyle, un groupe -O-alcényle, un groupe -O- aryle, un groupe -O-aralkyle, un groupe -O- aralcoxyalkyle, un groupe -O-alkylsulfonyle, un groupe -O-arylsulfonyle, un atome de chlore, un atome de brome ou un atome d'iode ;
R⁴ représente un groupe aryle ;
R⁵ représente un groupe alkyle, un groupe cycloalkyle, un groupe aryle, un groupe alcoxyalkyle ou un groupe hydroxyalkyle ;
et, où C* représente un atome de carbone asymétrique ;
b) éventuellement suivie par l'isolement du stéréoisomère souhaité ;
dans lequel le terme « alkyle » signifie un groupe alkyle linéaire ou ramifié seul ou en combinaison comprenant de 1 à 8 atomes de carbone ; et
le terme « aryle » signifie un groupe phényle ou naphtyle seul ou en combinaison qui peut être substitué par un ou plusieurs substituants choisis parmi les groupes alkyle, cycloalkyle, alcoxy, halogène, carboxy, alcoxycarbonyle, hydroxy, amino, nitro et trifluorométhyle ;
le terme « cycloalkyle » signifie un cycle cycloalkyle seul ou en combinaison ayant de 3 à 8 atomes de carbone et un cycle cycloalkyle préféré a de 3 à 6 atomes de carbone ;
le terme « alcényle » signifie un groupe alcényle ayant de 2 à 8 atomes de carbone ;
le terme « arylène » signifie un groupe phénylène ou naphtylène seul ou en combinaison qui peut être en outre substitué par un ou plusieurs substituants choisis parmi les groupes alkyle, cycloalkyle, halogène, nitro, alcoxy, hydroxy et amino.

2. Procédé selon la revendication 1, dans lequel R⁵ représente un groupe alkyle ou cycloalkyle.

3. Procédé selon la revendication 1 ou 2, dans lequel R⁴ représente un groupe phényle qui est éventuellement substitué par un ou plusieurs groupes indépendamment choisis parmi un groupe alkyle, un atome d'halogène ou un groupe nitro.

4. Procédé selon l'une quelconque des revendications 1 à 3, dans lequel R⁴ représente un groupe phényle et R⁵ représente un groupe méthyle.

5. Procédé selon l'une quelconque des revendications 1 à 4, dans lequel A représente un groupe phénylène et, dans lequel le groupe phénylène est éventuellement substitué par un à quatre substituants supplémentaires indépendamment choisis parmi un groupe alkyle, un atome d'halogène ou un groupe nitro.

6. Procédé selon l'une quelconque des revendications 1 à 5, dans lequel R² représente un groupe -O-benzyle ou un groupe -O-méthyle.

7. Procédé selon l'une quelconque des revendications 1 à 6, dans lequel un composé de formule 2 réagit avec un atome d'halogène ou un agent libérant un atome d'halogène.

8. Procédé selon la revendication 7, dans lequel le brome est utilisé.

9. Procédé selon l'une quelconque des revendications 1 à 8, dans lequel le stéréoisomère souhaité d'un composé de formule 1 ou d'un sel de celui-ci est isolé par cristallisation.

10. Procédé selon l'une quelconque des revendications 1 à 9, suivi par une réaction d'un alcoolate de métal, d'un amide de lithium ou d'un composé organolithium pour donner un composé de formule générale 5 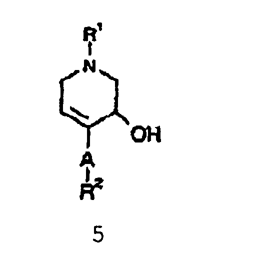 ou un sel de celui-ci.

11. Procédé selon l'une quelconque des revendications 1 à 10, dans lequel un composé de formule 1 ou un sel de celui-ci est converti en (3R,4S,5S)-4-[9-(3-benzyloxy-propoxy)-phényl]-5-éthoxy-3-(naphtalén-2-ylméthoxy)-pipéridine, **caractérisé en ce que**
a) le (1R,6R)-6-(4-benzyloxyphényl)-3-[(R)-1-phényl-éthyl]-7-oxa-3-aza-bicyclo[4.1.0]heptane est transformé en (3S)-4-(4-benzyloxyphényl)-1-[(1R)-phényl-éthyl]-1,2,3,6-tétrahydropyridin-3-ol par réaction avec du phényllithium ;
b) la réaction avec de l'hydrure de sodium et l'iodure d'éthyle donne la (3S)-4-(4-benzyloxyphényl)-3-éthoxy-1-[(1R)-phényléthyl] -1 , 2 , 3 , 6-tétrahydropyridine ;
c) l'hydroboration avec du borohydrure de sodium et de l'éthérate de trifluorure de bore suivie par la réaction avec de l'hydroxyde de potassium et du peroxyde d'hydrogène donne le (3R,4R,5S)-(4-benzyloxyphényl)-5-éthoxy-1-[(1R)-phényléthyl]-pipéridin-3-ol) ;
d) l'hydrogénolyse donne le (3R,4R,5S)-5-éthoxy-4-(4-hydroxyphényl)-pipéridin-3-ol ;
e) la réaction avec du dicarbonate de di-tert-butyle et de l'hydrogénocarbonate de sodium suivie par l'ajout de NaOH donne l'ester tert-butylique de l'acide (3R,4R,5S)-5-éthoxy-3-hydroxy-4-(4-hydroxyphényl)-pipéridine-1-carboxylique ;
f) le traitement avec du 3-bromopropoxy-méthylbenzène et du carbonate de potassium donne l'ester tert-butylique de l'acide (3R,4R,5S)-4-[4-(3-benzyloxypropoxy)-phényl]-5-éthoxy-3-hydroxypipéridine-1-carboxylique ;
g) la réaction avec du 2-bromométhylnaphtalène et de l'hydrure de sodium donne l'ester tert-butylique de l'acide (3R ,4S,5S)-4-[4-(3-benzyloxypropoxy)-phényl]-5-éthoxy-3-(naphtalén-2-ylméthoxy)-pipéridine-1-carboxylique ;
h) la réaction avec de l'acide chlorhydrique donne la (3R,4S,5S)-4-[4-(3-benzyloxypropoxy)-phényl]-5-éthoxy-3-(naphtalén-2-ylméthoxy)-pipéridine.

12. Composé selon la formule 1 ou un sel de celui-ci, dans lequel R¹, R², R⁴, R⁵ et A sont tels que définis selon l'une quelconque des revendications 1 à 6.

13. Composé selon la formule 2 ou un sel de celui-ci, dans lequel R¹, R², R⁴, R⁵ et A sont tels que définis selon l'une quelconque des revendications 1 à 6.

14. Composé selon la formule 5 ou un sel de celui-ci, dans lequel R¹, R², R⁴, R⁵ et A sont tels que définis selon l'une quelconque des revendications 1 à 6.

15. Composé selon l'une quelconque des revendications 12, 13 et 14 choisi parmi :
le (1R,6R)-6-(4-benzyloxyphényl)-3-[(R)-1-phényléthyl]-7-oxa-3-aza-bicyclo[4.1.0]heptane ;
la (R)-4-(4-benzyloxyphényl)-1-(1-phényléthyl)-1,2,3,6-tétrahydropyridine ;
le (R)-4-(4-benzyloxyphényl)-1-(1-phényléthyl)-pipéridin-4-ol ;
le (3S)-4-(4-benzyloxyphényl)-1-[(1R)-phényléthyl]-1,2,3,6-tétrahydropyridin-3-ol.

16. Utilisation d'un composé selon la revendication 12 dans la préparation d'inhibiteurs de la rénine.

17. Composé obtenu par le procédé selon l'une quelconque des revendications 1 à 10.
